# EUROPÄISCHE PATENTANMELDUNG

(11) **EP 2 400 412 A1**
(43) Veröffentlichungstag der Anmeldung: **28.12.2011**
(21) Anmeldenummer: 10167419.0
(22) Anmeldetag: 25.06.2010
(51) Int. Cl.: G06F 19/00

(54) **System und Verfahren zur Generierung maschinenlesbarer, patientenindividueller klinischer Behandlungspfade**

(71) Anmelder: Krallmann,, Herr Hermann, Prof.Dr., 14089 Berlin (DE)
(72) Erfinder: Krallmann,, Herr Hermann, Prof.Dr., 14089 Berlin (DE)
(74) Vertreter: Eisenführ, Speiser & Partner

(57) **Zusammenfassung**

Die Erfindung betrifft ein System zur Generierung maschinenlesbarer, patientenindividueller klinischer Behandlungspfade und ein Betriebsverfahren eines solchen Systems. Das System umfasst einen Komponentenspeicher umfassend eine Vielzahl von Komponentendatensätzen, wobei jeder Komponentendatensatz Informationen über einen Abschnitt eines Behandlungspfads beinhaltet, einen Regelspeicher umfassend eine Vielzahl von Regeldatensätzen, wobei jeder Regeldatensatz eine Regel repräsentiert, die eine oder mehrere Bedingungen zur Verknüpfbarkeit von Komponentendatensätzen beinhaltet, eine Patientendatenschnittstelle zum Empfangen von Patientendatensätzen aus einem Patientendatenspeicher, wobei ein Patientendatensatz mindestens eine Angabe zur Identifizierung eines Patienten oder mindestens eine Angabe zu einem Gesundheitszustand des Patienten umfasst, einen Behandlungspfadgenerator, der ausgebildet ist, in Abhängigkeit von über die Patientendatenschnittstelle empfangenen Pationtendatensätzen unter Anwendung einer oder mehrerer Regeln zwei oder mehrere der Komponentendatensätze auszuwählen und zu einem Behandlungspfad zu verknüpfen, mindestens eine Kontrolleinheit, die ausgebildet ist, die Erfüllung mindestens eines vordefinierten Prüfkriteriums zu überprüfen, wobei das Prüfkriterium das Vorhandensein eines Merkmals des generierten Behandlungspfads oder eines Merkmals eines Patientendatensatzes betrifft.

## Beschreibung

Die Erfindung betrifft ein System zur Generierung maschinenlesbarer, patientenindividueller klinischer Behandlungspfade.

Die Erfindung betrifft weiterhin ein Betriebsverfahren eines Systems zur Generierung maschinenlesbarer, patientenindividueller klinischer Behandlungspfade.

Die Erfindung liegt auf dem Gebiet der technischen, insbesondere der informationstechnischen, Unterstützung von Organisationen oder Organisationseinheiten im Bereich Gesundheitsversorgung, insbesondere in der Gesundheitsversorgung im klinischen Bereich, beispielsweise in Kliniken, Krankenhäusern, Hospitälern, Ärztehäusern oder anderen Behandlungszentren, in denen stationäre oder ambulante Behandlungen von Patienten durchgeführt werden. Unter Behandlung werden am Patienten vorgenommene Maßnahmen verstanden, beispielsweise Diagnose, Therapie, Überwachung oder Unfallversorgung.

Als klinischer Behandlungspfad wird die Definition, mit anderen Worten, die Darstellung oder Beschreibung einer Abfolge von Behandlungsschritten verstanden, die bei der Behandlung von Patienten durchgeführt werden können. Die einzelnen Behandlungsschritte können in einer Organisation bzw. Organisationseinheit oder verteilt über mehrere Organisationen bzw. Organisationseinheiten durchzuführen sein. Im Rahmen der vorliegenden Anmeldung wird neben dem Begriff "klinischer Behandlungspfad" der Kürze halber mit gleicher Bedeutung auch der Begriff "Behandlungspfad" verwendet..

Der Erfindung stellt ein System zur Generierung maschinenlesbarer, patientenindividueller klinischer Behandlungspfade bereit, umfassend
- einen Komponentenspeicher umfassend eine Vielzahl von maschinenlesbaren Komponentendatensätzen, wobei jeder Komponentendatensatz Informationen über einen Abschnitt eines klinischen Behandlungspfads beinhaltet,
- einen Regelspeicher umfassend eine Vielzahl von Regeldatensätzen, wobei jeder Regeldatensatz eine Regel repräsentiert, die eine oder mehrere Bedingungen zur Verknüpfbarkeit von Komponentendatensätzen beinhaltet,
- eine Patientendatenschnittstelle zum Empfangen von individuellen Patientendatensätzen aus einem externen Patientendatenspeicher, wobei ein Patientendatensatz mindestens eine Angabe zur Identifizierung eines Patienten und/oder mindestens eine Angabe zu einem Gesundheitszustand des Patienten umfasst,
- einen Behandlungspfadgenerator, der ausgebildet ist, in Abhängigkeit von über die Patientendatenschnittstelle empfangenen Patientendatensätzen unter Anwendung einer oder mehrerer Regeln zwei oder mehrere der Komponentendatensätze auszuwählen und zu einem maschinenlesbaren, patientenindividuellen klinischen Behandlungspfad zu verknüpfen,
- mindestens eine Kontrolleinheit, die ausgebildet ist, die Erfüllung mindestens eines vordefinierten Prüfkriteriums zu überprüfen, wobei das Prüfkriterium das Vorhandensein eines Merkmals des generierten klinischen Behandlungspfads und/oder eines Merkmals eines Patientendatensatzes betrifft.

Das erfindungsgemäße System wird vom Erfinder bzw. Anmelder auch als Wissensmanagementsystem für den Bereich Healthcare (Healthcare Knowledge Management System, HKMS) bzw. als Prototyp eines solchen Wissensmanagementsystems bezeichnet.

Die Durchführung von Diagnose- oder Behandlungsschritten ist nicht Teil des erfindungsgemäßen Verfahrens. Die Erfindung betrifft weder das Erstellen einer Diagnose für einen Patienten, also eine Untersuchung, das Sammeln von Daten und einen Vergleich der Daten mit Referenzen. Sie setzt die Existenz einer Diagnose voraus. Die Erfindung betrifft auch nicht die therapeutische Behandlung des Patienten. Die Erfindung befasst sich vielmehr mit technischen, insbesondere informationstechnischen, Mitteln und Verfahren zur Unterstützung von Unternehmensprozessen, die im klinischen Bereich nach einer Diagnose und vor einer Therapie anfallen. Insbesondere ermöglicht das erfindungsgemäße Verfahren eine automatisierte Ermittlung von möglichen Behandlungsschritten und deren Abbildung in einem maschinenlesbaren Behandlungspfad.

Die Erfindung beruht auf der Erkenntnis, dass in der Unternehmensorganisation im klinischen Umfeld das Problem besteht, dass sowohl die Komplexität hinsichtlich der Behandlungsmöglichkeiten, ihrer Indikationen, Kontraindikationen und Wechselwirkungen als auch die Anzahl von Patienten, die Menge und Komplexität der zu diesen Patienten verfügbaren Daten sowie die Häufigkeit von Mehrfacherkrankungen zunimmt. Gleichzeitig steigen sowohl Zeit- als auch Kostendruck im klinischen Umfeld. Unter diesen Rahmenbedingungen kommt es häufig zu Fehlern in der Vorbereitung und Planung von Behandlungen, mit negativen Auswirkungen auf die Behandlungsqualität.

Das erfindungsgemäße System hat den Vorteil, die Unternehmensorganisation im klinischen Umfeld, insbesondere die Planbarkeit von Prozessen im klinischen Umfeld, zu verbessern. Weiterhin hat das erfindungsgemäße System den Vorteil, Behandlungspfade auf patientenindividuelle Gegebenheiten anpassen zu können. Weiterhin hat das erfindungsgemäße System den Vorteil, Fehler in der Vorbereitung und Planung von Behandlungen zu vermeiden bzw. zu reduzieren oder zumindest frühzeitig zu erkennen.

Ein erfindungsgemäßes System ist in bevorzugten Ausführungsformen ein mit geeigneter Software eingerichtetes Computersystem oder eine Plattform. Als solches kann das erfindungsgemäße System als an einem Ort angeordnete Computervorrichtung oder als verteilte Computeranordnung mit an mehreren, räumlich getrennten Orten angeordneten und über ein Datennetz mit einander kommunizierenden Computervorrichtungen ausgebildet sein, die jeweils definierte Teil-Funktionen des erfindungsgemäßen Systems ausüben.

Ein mittels des Systems generierter individueller, maschinenlesbarer klinischer Behandlungspfad ist eine maschinenlesbare Repräsentation der bei einer Behandlung durchzuführenden Behandlungsschritte. Diese maschinenlesbare Repräsentation beinhaltet beispielsweise Informationen zu Art und Dauer von Behandlungsschritten, erforderlichen Ressourcen (materielle und personelle), Indikationen und Gegenanzeigen oder zu erwartenden Behandlungserfolgen.

Maschinenlesbar bedeutet in diesem Zusammenhang, dass ein klinischer Behandlungspfad von einer Maschine, beispielsweise einem Computer, lesbar und weiter verarbeitbar ist. Unmittelbar maschinenlesbar sind beispielsweise maschinenlesbar kodierte Symbole, Texte, Listen oder Datenrepräsentationen von Flussdiagrammen.

Das erfindungsgemäße System stellt Datenstrukturen und Technologien für eine maschinenlesbare Repräsentation und - wie noch ausgeführt wird - Adaption klinischer Behandlungspfade zur Verfügung.

Erfindungsgemäß wird ein klinischer Behandlungspfad aus zwei oder mehreren Abschnitten zusammengesetzt, wobei ein Abschnitt eine Abfolge von Behandlungsschritten repräsentiert, d.h. eine maschinenlesbare Darstellung bzw. Beschreibung einer Abfolge von Behandlungsschritten ist, die bei einer Behandlung von Patienten durchgeführt werden können. Ein erfindungsgemäß generierter Behandlungspfad weist somit eine modulare Struktur auf. Ein Abschnitt kann auch als Modul eines klinischen Behandlungspfads bezeichnet werden. Die in einem Abschnitt zusammengefassten Behandlungsschritte können zeitlich (beispielsweise nacheinander durchzuführende Behandlungsschritte), räumlich (beispielsweise am gleichen Ort durchzuführende Behandlungsschritte), oder aufgrund anderer Kriterien (beispielsweise ab einem bestimmten Patientenalter durchzuführende zusätzliche Behandlungsschritte) einander zugeordnet sein. Ein Abschnitt kann auch nur einen einzelnen Behandlungsschritt repräsentieren.

Informationen über einen solchen Abschnitt eines Behandlungspfads sind erfindungsgemäß als maschinenlesbarer Komponentendatensatz in einem Komponentenspeicher, der auch als Repository bezeichnet werden kann, abgelegt. Ein Komponentendatensatz definiert vorzugsweise die im betreffenden Abschnitt eines Behandlungspfads enthaltenen Behandlungsschritte, Indikationen, Gegenanzeigen, voraussichtliche Dauer und/oder voraussichtliche Wirkungen. Die Komponentendatensätze enthalten vorzugsweise klinisches Wissen in Form von Informationen über Abschnitte standardisierter Behandlungspfade. Während bei der Akquisition der Informationen über die standardisierten Behandlungspfade beispielsweise (erweiterte) ereignisgesteuerte Prozessketten (eEPKs) verwendet werden, werden diese im erfindungsgemäßen System im Komponentenspeicher als maschinenlesbare Repräsentation abgelegt, beispielsweise in Form der Business Process Modeling Notation (BPMN), die vom Behandlungspfadgenerator in einen Ausdruck der *Business* Process Execution Language (BPEL) übersetzt wird Eine Maschinenlesbarkeit in Form der Business Process Modeling Notation (BPMN) und der Business Process Execution Language (BPEL) hat gegenüber ontologie-basierten Repräsentationen von Behandlungspfaden den Vorteil einer besseren Performanz und Skalierbarkeit.

Die einzelnen Abschnitte eines klinischen Behandlungspfades werden erfindungsgemäß durch einen Behandlungspfadgenerator zu einem klinischen Behandlungspfad zusammengesetzt werden. Ein klinischer Behandlungspfad enthält zwei oder mehrere Abschnitte. Er kann einen seiner Abschnitte mehrfach enthalten. Dies verdeutlicht die modulare Struktur eines erfindungsgemäß generierten klinischen Behandlungspfades und den modularen Charakter der Komponentendatensätze.

Die Komponentendatensätze stehen im Komponentenspeicher dauerhaft zur Verfügung. Sie sind stets für eine neue Verknüpfung zu weiteren Behandlungspfaden wiederverwendbar. Die Komponentendatensätze können bei Bedarf geändert werden, beispielsweise, wenn neue Erkenntnisse über verbesserte Behandlungsschritte vorliegen.

Die mit dem erfindungsgemäßen System bzw. Verfahren generierten klinischen Behandlungspfade werden vorzugsweise in einem Behandlungspfadspeicher abgelegt, wobei in einer Ausführungsform jeweils eine Zuordnung zu den Patientendaten desjenigen Patienten erstellt wird, für den ein betreffender klinischer Behandlungspfad erstellt wird.

Der Behandlungspfadgenerator setzt zur Verknüpfung der Abschnitte Regeln ein. Die Regeln sind als Regeldatensätze in einem Regelspeicher, beispielsweise einer Business Rule Engine, abgelegt. Regelspeicher und Komponentenspeicher können als unterschiedliche Speicherbereiche eines gemeinsamen Speichers ausgebildet sein. Die Regeldatensätze enthalten Informationen darüber, welche Abschnitte unter welchen Bedingungen wie miteinander verknüpft werden können. Beispielsweise kann eine bestimmte Therapie eine weitere Anschlusstherapie zwingend oder optional nach sich ziehen oder andere Therapiemöglichkeiten ausschließen. Die in den Regeldatensätzen enthaltenen Informationen repräsentieren in Regeln abgebildetes klinisches Wissen darüber, wie standardisierte Behandlungspfade situationsabhängig (beispielsweise tageszeitabhängig oder in Abhängigkeit von der Kombinierbarkeit mit anderen Therapien) und in Abhängigkeit von patientenindividuellen Gegebenheiten, wie sie in den Patientendatensätzen enthalten sind, anzupassen sind oder wie Komponentendatensätze zu solchen situationsabhängigen und patientenindividuellen Behandlungspfaden verknüpft werden können. Die Informationen der Regeldatensätze sind vorzugsweise als Entscheidungstabellen erfasst.

Weiterhin verwendet der Behandlungspfadgenerator bei der Verknüpfung der Abschnitte zu Behandlungspfaden Patientendaten, die in Patientendatensätzen abgelegt sind. Ein Patientendatensatz enthält mindestens eine Identifizierungsangabe, wie beispielsweise Namen oder Geburtsdatum eines Patienten, und mindestens eine Angabe über den Gesundheitszustand des Patienten, beispielsweise eine Diagnose, eine Anamnese, einen Befund, einzelne Untersuchungsergebnisse, wie einen Blutwert, oder Angaben zu bisherigen Therapien. Diese Daten sind patientenindividuell, vorzugsweise existiert ein Patientendatensatz pro Patient. Durch Vergabe eindeutiger Identifikatoren können Patienten in Komponentendatensätzen abgespeicherte Behandlungspfadabschnitte zugeordnet werden. Vorzugsweise werden die Patientendaten durch eine Patientenontologie strukturiert und unter Anwendung ontologie-basierter Techniken verwaltet und bereitgestellt.

Die Patientendatensätze liegen meist in einem externen Patientendatenspeicher vor, so dass erfindungsgemäß eine Patientendatenschnittstelle vorgesehen ist, über die die Patientendaten empfangen werden können. Ein Ausführungsbeispiel sieht vor, dass das System einen weiteren Speicher aufweist und ausgebildet ist, einen Teil oder alle über die Patientendatenschnittstelle aus einem externen Patientendatenspeicher empfangenen individuellen Patientendaten in dem weiteren Speicher, zwischenzuspeichern oder dorthin zu kopieren. Dies ist insbesondere bevorzugt, um eine Unabhängigkeit von externen Speichern oder Systemen zu erreichen und/oder Datenverlusten vorzubeugen.

Abhängig von den Patientendaten werden vom Behandlungspfadgenerator zwei oder mehrere Abschnitte verknüpft. Beispielsweise kann in Abhängigkeit von der Diagnose und dem Alter sowie einer bestimmten Vortherapie eines Patienten eine erste Therapievariante (beispielsweise eine Operation) vom Behandlungspfadgenerator ausgewählt werden, wohingegen bei gleicher Diagnose und einem anderen Alter oder einer anderen Vortherapie eine zweite Therapievariante (beispielsweise eine Bestrahlung) ausgewählt wird. Auf diese Weise entstehen patientenindividuelle Behandlungspfade.

Erfindungsgemäß ist weiterhin vorgesehen, dass eine oder mehrere Kontrolleinheiten die Erfüllung mindestens eines vordefinierten Prüfkriteriums überprüft bzw. überprüfen. Die mindestens eine Kontrolleinheit ist vorzugsweise als intelligenter Software-Agent ausgebildet, der selbständig Kontrollen und Aktionen durchführen kann. Beispielsweise ist die Kontrolleinheit ausgebildet, Schwachstellen im generierten klinischen Behandlungspfad zu erkennen und automatisch zu kompensieren. Als Schwachstellen werden hier beispielsweise fehlende oder doppelte Behandlungsschritte, Fehler in der Reihenfolge von Behandlungsschritten, Ressourcendoppelbelegungen oder lange Wartezeiten zwischen Behandlungsschritten oder zwischen Abschnitten des Behandlungspfads verstanden.

Eine als Software-Agent ausgebildete Kontrolleinheit ist vorzugsweise als spezielle Art von Business Process Modeling Notation (BPMN)-Prozessen realisiert, die beispielsweise den Zustand des Patienten, allgemeine Qualitätsmerkmale oder zeitliche Anforderungen überwachen. Die Überprüfung und Überwachung erfolgt vorzugsweise nicht-invasiv, d.h. über eine Schnittstelle, beispielsweise eine Business Activity Monitoring (BAM)-Schnittstelle. Die Kontrolleinheit ist vorzugsweise ausgebildet, die generierten Behandlungspfade zu überwachen, indem sie - vorzugsweise iterativ - Daten erhebt, analysiert und regelbasiert Aktionen ableitet. Beispiele für solche Aktionen sind die Benachrichtigung von Verantwortlichen (z.B. per SMS) oder das Abbrechen eines laufenden Prozesses. Die - selbsttätige - Aktivität der Kontrolleinheit kann auf ein bestimmtes Gebiet (beispielsweise die Überwachung der Patientendaten oder die Anpassung an Klinikressourcen) beschränkt sein.

Vorzugsweise ist eine Kontrolleinheit mit einem Abschnitt, d.h. einem Komponentendatensatz verknüpft.

Das vordefinierte Prüfkriterium stellt dar, ob der generierte klinische Behandlungspfad oder die individuellen Patientendaten ein bestimmtes Merkmal aufweisen oder nicht. Beispiele für durch das Prüfkriterium abzuprüfende Merkmale des klinischen Behandlungspfads oder der individuellen Patientendaten sind Abschlusskontrollen durch einen Arzt vor der Entlassung des Patienten, Mindest- oder Maximalzeitabstände zwischen Behandlungen, Verträglichkeit von einzusetzenden Medikamenten bei Allergien oder Gegenanzeigen oder allgemeine Qualitätsmerkmale, wie sie beispielsweise in Key Performance indicators (KPls) festgelegt werden.

Erfindungsgemäß ist es somit nicht nur möglich, auf Basis von patientenindividuellen Daten und in Form von Komponentendatensätzen und Regeldatensätzen abgespeichertem klinischen Prozesswissen maschinenlesbare, patientenindividuelle klinische Behandlungspfade zu generieren, sondern diese Behandlungspfade und/oder die Patientendaten zu überprüfen und somit eine erhöhte Qualität der generierten Behandlungspfade sicherzustellen.

In einer Fortbildungsform weist das System einen weiteren Wissensspeicher auf, in dem in maschinenlesbarer Form behandlungsbezogene Informationen abgelegt sind. Diese behandlungsbezogenen Informationen sind vorzugsweise durch Ontologien repräsentiert und enthalten Angaben zu Behandlungsfällen und für die Veränderung oder Adaption von klinischen Behandlungspfaden. Eine Ontologie ist eine sprachlich gefasste und formal geordnete Darstellung einer Menge von Begrifflichkeiten und der zwischen ihnen bestehenden Beziehungen in einem bestimmten Gegenstandsbereich und dient dazu, Wissen in digitalisierter und formaler Form verfügbar zu machen. Auf diesen Wissensspeicher können vorzugsweise der Behandlungspfadgenerator oder die Kontrolleinheit zugreifen und die im Wissensspeicher abgelegten Informationen für die Auswahl und Verknüpfung der Komponentendatensätze oder der Überprüfung der Erfüllung des Prüfkriteriums einsetzen. Regelspeicher, Komponentenspeicher und Wissensspeicher können als unterschiedliche Speicherbereiche eines gemeinsamen Speichers ausgebildet sein.

Die Erfassung der in einem Komponentendatensatz oder in einem Regeldatensatz enthaltenen Informationen, die im Stand der Technik heute noch teils als tangibles und teils als intangibles Wissen vorliegen, ist mit erheblichem Aufwand verbunden. In einer Fortbildung der vorliegenden Erfindung ist es daher vorgesehen, zusätzlich eine Erfassung von Komponenten- oder Regeldatensätzen durchzuführen. Dies erfolgt vorzugsweise automatisiert anhand einer Auswertung ausgewählter Informationen, wie behandlungsbezogenes klinisches Wissen oder standardisierte klinische Behandlungspfade. Wo intangibles Wissen nicht automatisch, sondern nur durch Befragung der Wissensträger erfasst werden kann, wird vorzugsweise zumindest das erfassbare tangible Wissen automatisiert eingebunden, so dass eine teilautomatisiert Erfassung durchgeführt wird. Dazu werden beispielsweise Technologien wie Data Mining und Data Warehouse eingesetzt.

Die Erfindung kann dadurch fortgebildet werden, dass die Patientendatenschnittstelle ausgebildet ist, individuelle Patientendatensätze aus einem externen Patientendatenspeicher wiederholt zu empfangen, wobei das wiederholte Empfangen in unterschiedlichen Varianten kontinuierlich, nach vorbestimmten Zeitabschnitten oder an bestimmten Stellen des generierten Behandlungspfads erfolgt und/oder von einem Nutzer initiiert wird. Bevorzugt ist es, die Patientendatensätze nicht nur einmalig, sondern mehrmals zu empfangen und dem Behandlungspfadgenerator und der Kontrolleinheit somit jeweils aktualisierte Patientendaten zur Verfügung zu stellen. Dies ist insbesondere deshalb bevorzugt, weil die Patientendaten von in Behandlung befindlichen Patienten, vor allem die den Gesundheitszustand betreffenden Patientendaten, Veränderungen unterliegen und durch die bevorzugte Fortbildungsform die Qualität des generierten Behandlungspfads durch die Verwendung aktueller individueller Patientendatensätze gesteigert werden kann. Bevorzugte Empfangsintervalle können je nach Fallkonstellation unterschiedlich sein: von einem kontinuierlichen Empfangen mit sehr kurz getakteten Aktualisierungen bis hin zu Aktualisierungen nach vorbestimmten Zeitabschnitten, beispielsweise täglich oder nach bestimmten Abschnitten oder Teilabschnitten des Behandlungspfades, beispielsweise nach einem Eingriff oder einer bestimmten Therapiemaßnahme, oder durch Eingriff bzw. Auslösung des Empfangens durch einen Nutzer des Systems. Weiterhin kann das wiederholte Empfangen von Patientendatensätzen auch durch ein Ereignis, beispielsweise das Eintreffen eines Spenderorgans, ausgelöst werden. Vorzugsweise weist das System eine entsprechende weitere Schnittstelle zum Empfangen von Informationen über solche Ereignisse auf.

Die Erfindung kann dadurch fortgebildet werden, dass die Kontrolleinheit ausgebildet ist, die Erfüllung mindestens eines vordefinierten Prüfkriteriums wiederholt zu überprüfen, wobei das wiederholte Überprüfen vorzugsweise kontinuierlich, nach vorbestimmten Zeitabschnitten oder an bestimmten Stellen des generierten Behandlungspfads erfolgt und/oder von einem Nutzer initiiert wird.

Bevorzugt ist es, dass die Erfüllung mindestens eines vordefinierten Prüfkriteriums nicht nur einmalig, sondern mehrmals überprüft wird. Auf diese Weise kann Veränderungen Rechnung getragen werden. Überprüfungsintervalle können je nach Fallkonstellation unterschiedlich sein: von einem kontinuierlichen, sehr kurz getakteten Überprüfen bis zu einem Überprüfen nach vorbestimmten Zeitabschnitten, beispielsweise täglich oder nach bestimmten Abschnitten oder Teilabschnitten des Behandlungspfades, etwa nach einem Eingriff oder einer bestimmten Therapiemaßnahme, oder durch Eingriff bzw. Auslösung des Überprüfen durch einen Nutzer des Systems. Weiterhin kann das wiederholte Überprüfen von Patientendatensätzen auch durch ein Ereignis, beispielsweise das Eintreffen eines Spenderorgans, ausgelöst werden. Vorzugsweise weist das System eine entsprechende weitere Schnittstelle zum Empfangen von Informationen über solche Ereignisse auf. Die wiederholte Überprüfung der Erfüllung mindestens eines vordefinierten Prüfkriteriums ist insbesondere in Kombination mit einem wiederholten Empfangen von individuellen Patientendatensätzen bevorzugt, da durch die wiederholte Überprüfung aktualisierter Patientendaten die Qualität des generierten Behandlungspfads in besonders vorteilhafter Weise verbessert werden kann.

Die Erfindung kann dadurch fortgebildet werden, dass die Kontrolleinheit ausgebildet ist, in Abhängigkeit von einem Ergebnis der Überprüfung den Behandlungspfad zu verändern und/oder ein Warnsignal auszugeben.

Diese Fortbildungsfiorm ermöglicht es, dass die Kontrolleinheit - vorzugsweise selbsttätig - den Behandlungspfad beispielsweise durch Austausch, Ergänzung oder Entfernung eines oder mehrerer Abschnitte oder durch Terminierung oder Neustart eines oder mehrerer Prozesse verändert. Auf diese Weise kann der Behandlungspfad angepasst werden, wenn die Überprüfung beispielsweise einen Fehler ergeben hat oder einer Wiederholung der Überprüfung aktualisierte Patientendaten zugrunde gelegen haben, die einen anderen als den ursprünglich vorgesehenen Behandlungspfad oder Abschnitt erfordern oder begünstigen. Auf diese Weise kann der Behandlungspfad auf aktuelle Veränderungen individueller Patienten angepasst werden, ohne dass Eingriffe von Nutzern notwendig sind. Das Ausgeben eines Warnsignals umfasst auch das Versenden von Warnungen, beispielsweise an bestimmte Nutzer oder Nutzergruppen.

Diese Fortbildungsform ermöglicht eine automatische Adaption von standardisierten klinischen Behandlungspfaden an die Eigenschaften des konkreten Behandlungsfalls im Sinne einer Überwachung und Optimierung von patientenindividuellen Behandlungspfaden.

Die Veränderung des Behandlungspfads auf der Ebene der Abschnitte kann eine Anpassung des Behandlungspfads durch Austauschen, Entfernen oder Ergänzen von ganzen Abschnitten beinhalten, ohne dass die einzelnen, in einem Abschnitt zusammengefassten Behandlungsschritte verändert werden müssen.

Bei einer Variante ist die Kontrolleinheit ausgebildet, vor einer Veränderung des Behandlungspfads die Freigabe durch einen Nutzer mit einer bestimmten Qualifikation anzufordern und die Veränderung des Behandlungspfads nur nach erfolgter Freigabe vorzunehmen. Dadurch wird sichergestellt, dass bei einer Veränderung des Behandlungspfads durch die Kontrolleinheit, ggf. beschränkt auf bestimmten Arten von Veränderungen, eine Freigabe des geänderten Behandlungspfad durch einen Nutzer mit einer bestimmten Qualifikation erfolgen muss. Dadurch kann eine Kontrolle der von der Kontrolleinheit automatisch initiierten und durchgeführten Veränderungen sichergestellt werden.

Die Erfindung kann dadurch fortgebildet werden, dass die Kontrolleinheit ausgebildet ist, in Abhängigkeit von einem Ergebnis der Überprüfung einen Regeldatensatz und/oder einen Komponentendatensatz zu verändern.

Alternativ oder zusätzlich kann vorgesehen sein, dass die Kontrolleinheit die zur Generierung der Behandlungspfade genutzten Regel- und/oder Komponentendatensätze verändert. Dies kann beispielsweise wünschenswert sein, wenn durch wiederholtes Auftreten von bestimmten Überprüfungsergebnissen auf einen Fehler oder eine verbesserungsfähige Information in den Komponentendatensätzen oder den Regeldatensätzen geschlossen werden kann. Auf diese Weise können die der Generierung der klinischen Behandlungspfade zugrundliegenden Daten kontinuierlich verbessert werden. Dabei ist es bevorzugt, dass eine Freigabe eines geänderten Regel- und/oder Komponentendatensatzes durch einen Nutzer mit einer bestimmten Qualifikation erfolgen muss. Dadurch kann eine Kontrolle der von der Kontrolleinheit automatisch initiierten und durchgeführten Veränderungen sichergestellt werden.

Die Erfindung kann dadurch fortgebildet werden, dass der Behandlungspfadgenerator ausgebildet ist, in Abhängigkeit von einem Ergebnis der Überprüfung den Behandlungspfad zu verändern und/oder ein Warnsignal auszugeben.

Alternativ oder zusätzlich zu der oben beschriebenen Veränderung des Behandlungspfads und/oder Ausgabe eines Warnsignals in Abhängigkeit von einem Ergebnis der Überprüfung durch die Kontrolleinheit kann die Veränderung des Behandlungspfads und/oder Ausgabe eines Warnsignals in Abhängigkeit von einem Ergebnis der Überprüfung auch durch den Behandlungspfadgenerator erfolgen.

Die Erfindung kann dadurch fortgebildet werden, dass der Behandlungspfadgenerator ausgebildet ist, in Abhängigkeit von einem Ergebnis der Überprüfung einen Regeldatensatz und/oder einen Komponentendatensatz zu verändern.

Alternativ oder zusätzlich zu der oben beschriebenen Veränderung der Regeldatensätze und/oder der Komponentendatensätze in Abhängigkeit von einem Ergebnis der Überprüfung durch die Kontrolleinheit kann die Veränderung der Regeldatensätze und/oder der Komponentendatensätze Abhängigkeit von einem Ergebnis der Überprüfung auch durch den Behandlungspfadgenerator erfolgen.

Eine bevorzugte Fortbildungsform der Erfindung ist gekennzeichnet durch eine Ressourcenschnittstelle zum Empfangen von Ressourcendatensätzen aus einem weiteren klinischen System, wobei die Ressourcendatensätze Informationen über die Verfügbarkeit von Klinikressourcen beinhalten, und wobei der Behandlungspfadgenerator ausgebildet ist, in Abhängigkeit von den Ressourcendatensätzen zwei oder mehrere der Komponentendatensätze aus einem Komponentenspeicher auszuwählen und/oder das mindestens eine vordefinierte Prüfkriterium das Vorhandensein eines Merkmals eines Ressourcendatensatzes betrifft.

Diese Fortbildungsform sieht es vor, die Verfügbarkeit von Klinikressourcen in die Generierung der Behandlungspfade mit einzubeziehen. Klinikressourcen können beispielsweise Personal, Qualifikationen, Räumlichkeiten, Material, Instrumente oder medizinische Geräte sowie deren Belegung durch Patienten sein. Dabei ist es besonders bevorzugt, wenn auch die Ressourcendatensätze wiederholt empfangen werden und somit aktuelle Ressourcendatensätze zur Verfügung stehen. Bevorzugte 1=mpfangsintervalle können unterschiedlich sein: von einem kontinuierlichen Empfangen mit sehr kurz getakteten Aktualisierungen zu Aktualisierungen nach vorbestimmten Zeitabschnitten, beispielsweise täglich oder vor oder nach bestimmten Abschnitten oder Teilabschnitten des Behandlungspfades, beispielsweise vor einem Eingriff oder einer bestimmten Therapiemaßnahme mit besonderen Ressourcenanforderungen, oder durch Eingriff bzw. Auslösung des Empfangens durch einen Nutzer des Systems. Weiterhin kann das wiederholte Empfangen von Ressourcendatensätzen auch durch ein Ereignis, beispielsweise das Eintreffen eines Spenderorgans, ausgelöst werden. Vorzugsweise weist das System eine entsprechende weitere Schnittstelle zum Empfangen von Informationen über solche Ereignisse auf.

Ein Ausführungsbeispiel sieht vor, dass das System einen weiteren Speicher aufweist und ausgebildet ist, einen Teil oder alle über die Ressourcenschnittstelle aus einem weiteren klinischen System empfangenen Ressourcendatensätze in dem weiteren Speicher, zwischenzuspeichern oder dorthin zu kopieren. Dies ist insbesondere bevorzugt, um eine Unabhängigkeit von externen Speichern oder Systemen zu erreichen oder Datenverlusten vorzubeugen.

Eine weitere bevorzugte Fortbildungsform der Erfindung ist gekennzeichnet durch einen Ergebnisspeicher umfassend eine Vielzahl von Ergebnisdatensätzen, wobei jeder Ergebnisdatensatz Informationen über Kosten und/oder Dauer der Behandlungsschritte und/oder Informationen über Veränderungen von Patientendaten umfasst.

Diese bevorzugte Fortbildungsform ermöglicht es, im Anschluss an die Behandlung eines Patienten eine vereinfachte Abrechnung der Leistungen und/oder eine vereinfachte Leistungs- bzw. Qualitätskontrolle durchzuführen, da in den Ergebnisdatensätzen patientenindividuelle Informationen zu den Kosten und/oder der Dauer der Behandlung und/oder den Auswirkungen der Behandlung auf den Gesundheitszustand des Patienten hinterlegt sind. Weiterhin stellen die in den Ergebnisdatensätzen gespeicherten Informationen eine Wissensquelle dar, die wiederum als Basis zur Verbesserung der Komponentendatensätze oder der Regeldatensätze genutzt werden kann. Dazu ist es bevorzugt, die Ergebnisdatensätze zu analysieren, beispielsweise durch (Reporting, Data Mining oder Data Warehousing, und die gewonnenen Erkenntnisse in den Komponentenspeicher, den Regelspeicher oder den Wissensspeicher einzupflegen.

Weiterhin ist es bevorzugt, dass das System eine Benutzerschnittstelle aufweist und ausgebildet ist, vor einer Veränderung von Komponentendatensätzen, Regeldatensätzen, Patientendatensätzen, Ressourcendatensätzen und/oder Ergebnisdatensätzen eine Freigabe durch bestimmte Nutzer und/oder bestimmte Nutzergruppen über die Benutzerschnittstelle anzufordern. Diese Fortbildungsform hat den Vorteil, dass definierte Personen und/oder der Personengruppen, auch als Rollen bezeichnet, beispielsweise Ärzte, Oberärzte, Pflegepersonal, bestimmte Daten im System verändern, d.h. beispielsweise auch ergänzen, können. Die Veränderung von Komponentendatensätzen, Regeldatensätzen, Patientendatensätzen, Ressourcendatensätzen und/oder Ergebnisdatensätzen erfolgt vorzugsweise erst nach Erhalt der angeforderten Freigabe. Eine weitere Fortbildungsform sieht vor, dass das System ausgebildet ist, für bestimmte Nutzer und/oder bestimmte Nutzergruppen nur bestimmte Teile eines generierten Behandlungspfads über die Benutzerschnittstelle auszugeben oder anzuzeigen. Dies hat den Vorteil, dass Anpassungen nur in Abhängigkeit von bestimmten Rollen vorgenommen werden können und die Informationsdichte für Nutzer reduziert und damit die Aufnahmefähigkeit der Informationen beim Nutzer erleichtert werden kann, da nur für den jeweiligen Nutzer bzw. die jeweilige Nutzergruppen relevante Informationen angezeigt oder ausgegeben werden. Weiterhin ist es bevorzugt, dass das System ausgebildet ist, das Auswählen von zwei oder mehreren Komponentendatensätzen, das Verknüpfen der ausgewählten Komponentendatensätze zu einem Behandlungspfad, das Verändern des Behandlungspfads und/oder Ausgeben eines Warnsignals und/oder das Verändern der Regeldatensätze und/oder der Komponentendatensätze wiederholt durchzuführen, wobei die Wiederholung vorzugsweise kontinuierlich, nach vorbestimmten Zeitabschnitten oder an bestimmten Stellen des generierten Behandlungspfads erfolgt oder von einem Nutzer initiiert wird.

Ein weiterer Aspekt der Erfindung ist ein eingangs genanntes Betriebsverfahren eines Systems zur Generierung patientenindividueller klinischer Behandlungspfade, umfassend die Schritte:
- Empfangen von individuellen Patientendaten aus einem externen Patientendatenspeicher, wobei ein Patientendatensatz mindestens eine Angabe zur Identifizierung eines Patienten und/oder mindestens eine Angabe zu einem Gesundheitszustand des Patienten umfasst,
- Auswählen von zwei oder mehreren der Komponentendatensätze aus einem Komponentenspeicher, der eine Vielzahl von maschinenlesbaren Komponentendatensätzen umfasst, wobei jeder Komponentendatensatz Informationen über einen Abschnitt eines klinischen Behandlungspfads beinhaltet, wobei das Auswählen in Abhängigkeit von den Patientendatensätzen und unter Anwendung einer oder mehrerer Regeln erfolgt, die jeweils eine oder mehrere Bedingungen zur Verknüpfbarkeit von Komponentendatensätzen beinhalten und durch jeweils einen Regeldatensatz aus einem Regelspeicher repräsentiert werden,
- Verknüpfen der ausgewählten Komponentendatensätze zu einem maschinenlesbaren, patientenindividuellen klinischen Behandlungspfad,
- Überprüfen der Erfüllung mindestens eines vordefinierten Prüfkriteriums, wobei das Prüfkriterium das Vorhandensein eines Merkmals des generierten klinischen Behandlungspfads und/oder eines Patientendatensatzes betrifft.

Das erfindungsgemäße Verfahren kann fortgebildet werden durch den Schritt: Wiederholtes Empfangen von individuellen Patientendatensätzen aus einem externen Patientendatenspeicher, wobei die Wiederholung vorzugsweise kontinuierlich, nach vorbestimmten Zeitabschnitten oder an bestimmten Stellen des generierten Behandlungspfads erfolgt oder von einem Nutzer initiiert wird.

Das Verfahren kann weiterhin fortgebildet werden durch den Schritt: Wiederholtes Überprüfen der Erfüllung mindestens eines vordefinierten Prüfkriteriums, wobei die Wiederholung vorzugsweise kontinuierlich, nach vorbestimmten Zeitabschnitten oder an bestimmten Stellen des generierten Behandlungspfads erfolgt oder von einem Nutzer initiiert wird.

Das Verfahren kann weiterhin fortgebildet werden durch den Schritt: Verändern des Behandlungspfads oder Ausgeben eines Warnsignals in Abhängigkeit von einem Ergebnis der Überprüfung.

Das Verfahren kann weiterhin fortgebildet werden durch den Schritt: Verändern eines Regeldatensatzes oder eines Komponentendatensatzes in Abhängigkeit von einem Ergebnis der Überprüfung.

Das Verfahren kann weiterhin fortgebildet werden durch den Schritt: Empfangen von Ressourcendatensätzen aus einem weiteren klinischen System, wobei die Ressourcendatensätze Informationen über die Verfügbarkeit von Klinikressourcen beinhalten, und wobei der Schritt des Auswählens von zwei oder mehreren der Komponentendatensätze aus einem Komponentenspeicher in Abhängigkeit von den Ressourcendatensätzen erfolgt oder mindestens ein Prüfkriterium das Vorhandensein eines Merkmals eines Ressourcendatensatzes betrifft.

Das Verfahren kann weiterhin fortgebildet werden durch den Schritt: Speichern von Ergebnisdatensätzen über die bei der Durchführung der in generierten Behandiungspfaden repräsentierten Behandlungsschritte, wobei die Ergebnisdatensätze Informationen über Kosten oder Dauer der Behandlungsschritte oder Informationen über Veränderungen von Patientendaten umfasst.

Diese fortgebildeten Verfahren weisen Merkmale bzw. Verfahrensschritte auf, die ein erfindungsgemäßes System oder seine oben beschriebenen Fortbildungen durchführen. Zu den Ausführungsformen, spezifischen Merkmalen, Varianten und Vorteilen der Merkmale dieses Verfahrens und der Verfahrensfortbildungen wird daher auf die vorangegangene Beschreibung zu den entsprechenden Systemmerkmalen verwiesen.

Eine bevorzugte Ausführungsform der Erfindung wird beispielhaft anhand der beiliegenden Figuren beschrieben. Es zeigen:
- Fig. 1:: Eine schematische Darstellung einer bevorzugten Ausführungsform eines erfindungsgemäßen Systems und
- Fig. 2:: eine schematische Darstellung einer bevorzugten Ausführungsform eines erfindungsgemäßen Betriebsverfahrens.

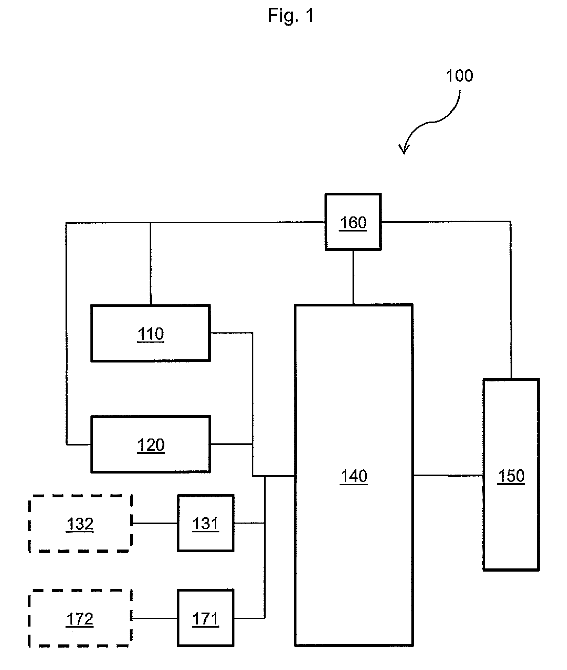

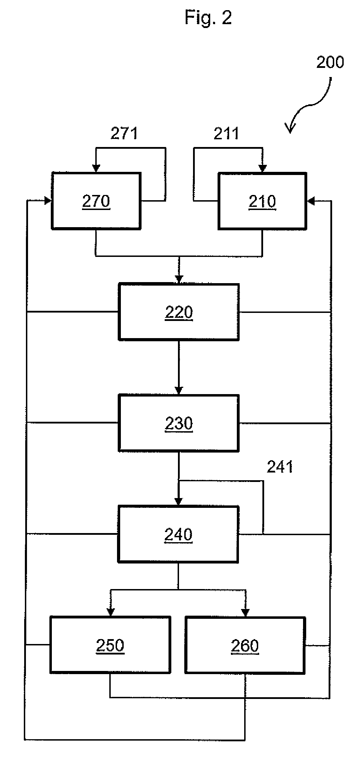

Figur 1 stellt eine bevorzugte Ausführungsform eines erfindungsgemäßen Systems 100 zur Generierung maschinenlesbarer, patientenindividueller klinischer Behandlungspfade in schematischer Ansicht dar, das bevorzugt nach der in Figur 2 dargestellten bevorzugten Ausführungsform eines erfindungsgemäßen Betriebsverfahrens 200 betrieben werden kann.

Das erfindungsgemäße System 100 zur Generierung maschinenlesbarer, patientenindividueller klinischer Behandlungspfade gemäß Figur 1 umfasst einen Behandlungspfadgenerator 140, einen Komponentenspeicher 110, einen Regelspeicher 120 und einen Behandlungspfadspeicher 150. Weiterhin umfasst das System 100 eine Patientendatenschnittstelle 131 zum Empfangen von individuellen Patientendatensätzen aus einem externen Patientendatenspeicher 132 und eine Ressourcenschnittstelle 171 zum Empfangen von Ressourcendatensätzen aus einem weiteren klinischen System 172. Schließlich umfasst das System 100 auch eine Kontrolleinheit 160.

Die Bestandteile des Systems 100 können als an einem Ort angeordnete Computervorrichtung oder als verteilte Computeranordnung mit an mehreren, räumlich getrennten Orten angeordneten und über ein Datennetz mit einander kommunizierenden Computervorrichtungen ausgebildet sein.

Die Kontrolleinheit 160 ist vorzugsweise als intelligenter Software-Agent ausgebildet, der selbsttätig agieren kann. Der Software-Agent ist vorzugsweise als spezielle Art von Business Process Modeling Notation (BPMN)-Prozessen realisiert, die beispielsweise den Zustand des Patienten, allgemeine Qualitätsmerkmale oder zeitliche Anforderungen überwachen. Die Komponenten-, Regel- und Behandlungspfadspeicher 110, 120, 150 können als separate Speicher oder als Speicherbereiche eines gemeinsamen Speichers ausgebildet sein. Der externe Patientendatenspeicher 132 und das weitere klinische System 172 sind nicht Teil des erfindungsgemäßen Systems 100. Das erfindungsgemäße System kann aber vorzugsweise weitere Speicher oder Speicherbereiche aufweisen, in denen Patientendatensätze und/oder Ressourcendatensätze zwischengespeichert oder kopiert werden können.

Der Betrieb des Systems 100 erfolgt vorzugsweise nach dem in Figur 2 dargestellten Verfahren 200 mit den folgenden Schritten: Empfangen 210 von individuellen Patientendatensätzen aus einem externen Patientendatenspeicher oder ggf. aus einem systeminternen Zwischenspeicher, Empfangen 270 von Ressourcendatensätzen aus einem weiteren klinischen System oder ggf. aus einem systeminternen Zwischenspeicher, Auswählen 220 von zwei oder mehreren Komponentendatensätzen aus einem Komponentenspeicher. Das Auswählen 220 erfolgt in Abhängigkeit von den Patientendatensätzen und den Ressourcendatensätzen und unter Anwendung einer oder mehrerer Regeln, die als Regeldatensätze im Regelspeicher 120 abgelegt sind. Die ausgewählten Komponentendatensätze werden im Schritt 230 zu einem maschinenlesbaren, patientenindividuellen klinischen Behandlungspfad verknüpft. Als weiteren Schritt umfasst das Betriebsverfahren 200 das Überprüfen 240 der Erfüllung mindestens eines vordefinierten Prüfkriteriums, wobei das Prüfkriterium das Vorhandensein eines Merkmals des generierten klinischen Behandlungspfads, eines Patientendatensatzes oder eines Ressourcendatensatzes betrifft. In Abhängigkeit von einem Ergebnis der Überprüfung wird im Schritt 250 der Behandlungspfad verändert und/oder ein Warnsignal ausgegeben und/oder im Schritt 260 die Regeldatensätze und/oder die Komponentendatensätze verändert.

Das Empfangen 210 von individuellen Patientendatensätzen läuft vorzugsweise wiederholt ab, wie durch Pfeil 211 angedeutet. Auch das Empfangen 270 von Ressourcendatensätzen läuft vorzugsweise wiederholt ab, wie durch Pfeil 271 angedeutet, ebenso wie das Überprüfen 240, siehe Pfeil 241.

Auch die übrigen Schritte - Auswählen 220 von zwei oder mehreren Komponentendatensätzen, Verknüpfen 230 der ausgewählten Komponentendatensätze zu einem Behandlungspfad, Verändern 250 des Behandlungspfads und/oder Ausgeben eines Warnsignals und/oder Verändern 260 der Regeldatensätze und/oder der Komponentendatensätze - können vorzugsweise wiederholt ablaufen, wobei die Wiederholung vorzugsweise kontinuierlich, nach vorbestimmten Zeitabschnitten oder an bestimmten Stellen des generierten Behandlungspfads erfolgt oder von einem Nutzer initiiert wird. Auch das Verfahren 200 kann vorzugsweise insgesamt oder in Teilabschnitten wiederholt werden, wie in Figur 2 durch die nicht beschrifteten Pfeile angedeutet, wobei auch hier die Wiederholung vorzugsweise kontinuierlich, nach vorbestimmten Zeitabschnitten oder an bestimmten Stellen des generierten Behandlungspfads erfolgt oder von einem Nutzer initiiert wird.

## Patentansprüche

1. System (100) zur Generierung maschinenlesbarer, patientenindividueller klinischer Behandlungspfade, umfassend
- einen Komponentenspeicher (110) umfassend eine Vielzahl von maschinenlesbaren Komponentendatensätzen, wobei jeder Komponentendatensatz Informationen über einen Abschnitt eines klinischen Behandlungspfads beinhaltet,
- einen Regelspeicher (120) umfassend eine Vielzahl von Regeldatensätzen, wobei jeder Regeldatensatz eine Regel repräsentiert, die eine oder mehrere Bedingungen zur Verknüpfbarkeit von Komponentendatensätzen beinhaltet,
- eine Patientendatenschnittstelle (131) zum Empfangen von individuellen Patientendatensätzen aus einem externen Patientendatenspeicher (132), wobei ein Patientendatensatz mindestens eine Angabe zur Identifizierung eines Patienten und mindestens eine Angabe zu einem Gesundheitszustand des Patienten umfasst,
- einen Behandlungspfadgenerator (140), der ausgebildet ist, in Abhängigkeit von über die Patientendatenschnittstelle empfangenen Patientendatensätzen unter Anwendung einer oder mehrerer Regeln zwei oder mehrere der Komponentendatensätze auszuwählen und zu einem maschinenlesbaren, patientenindividuellen klinischen Behandlungspfad zu verknüpfen,
- mindestens eine Kontrolleinheit (160), die ausgebildet ist, die Erfüllung mindestens eines vordefinierten Prüfkriteriums zu überprüfen, wobei das Prüfkriterium das Vorhandensein eines Merkmals des generierten klinischen Behandlungspfads oder eines Merkmals eines Patientendatensatzes betrifft.

2. System (100) nach dem vorhergehenden Anspruch,
**dadurch gekennzeichnet, dass** die Patientendatenschnittstelle (131) ausgebildet ist, individuelle Patientendatensätze aus einem externen Patientendatenspeicher (132) wiederholt zu empfangen, wobei das wiederholte Empfangen vorzugsweise kontinuierlich, nach vorbestimmten Zeitabschnitten oder an bestimmten Stellen des generierten Behandlungspfads erfolgt oder von einem Nutzer initiiert wird.

3. System (100) nach einem der vorhergehenden Ansprüche,
**dadurch gekennzeichnet, dass** die Kontrolleinheit (160) ausgebildet ist, die Erfüllung mindestens eines vordefinierten Prüfkriteriums wiederholt zu überprüfen, wobei das wiederholte Überprüfen vorzugsweise kontinuierlich, nach vorbestimmten Zeitabschnitten oder an bestimmten Stellen des generierten Behandlungspfads erfolgt oder von einem Nutzer initiiert wird.

4. System (100) nach einem der vorhergehenden Ansprüche,
**dadurch gekennzeichnet, dass** die Kontrolleinheit (160) ausgebildet ist, in Abhängigkeit von einem Ergebnis der Überprüfung den Behandlungspfad zu verändern oder ein Warnsignal auszugeben.

5. System (100) nach einem der vorhergehenden Ansprüche,
**dadurch gekennzeichnet, dass** die Kontrolleinheit (160) ausgebildet ist, in Abhängigkeit von einem Ergebnis der Überprüfung einen Regeldatensatz oder einen Komponentendatensatz zu verändern.

6. System (100) nach einem der vorhergehenden Ansprüche,
**dadurch gekennzeichnet, dass** der Behandlungspfadgenerator (140) ausgebildet ist, in Abhängigkeit von einem Ergebnis der Überprüfung den Behandlungspfad zu verändern oder ein Warnsignal auszugeben.

7. System (100) nach einem der vorhergehenden Ansprüche,
**gekennzeichnet durch** eine Ressourcenschnittstelle (171) zum Empfangen von Ressourcendatensätzen aus einem weiteren klinischen System (172), wobei die Ressourcendatensätze Informationen über die Verfügbarkeit von Klinikressourcen beinhalten,
und wobei der Behandlungspfadgenerator (140) ausgebildet ist, in Abhängigkeit von den Ressourcendatensätzen zwei oder mehrere der Komponentendatensätze aus einem Komponentenspeicher (110) auszuwählen oder das mindestens eine vordefinierte Prüfkriterium das Vorhandensein eines Merkmals eines Ressourcendatensatzes betrifft.

8. System (100) nach einem der vorhergehenden Ansprüche,
**gekennzeichnet durch** einen Ergebnisspeicher umfassend eine Vielzahl von Ergebnisdatensätzen, wobei jeder Ergebnisdatensatz Informationen über Kosten oder Dauer der Behandlungsschritte oder Informationen über Veränderungen von Patientendaten umfasst.

9. Betriebsverfahren (200) eines Systems zur Generierung maschinenlesbarer, patientenindividueller klinischer Behandlungspfade,
umfassend die Schritte:
- Empfangen (210) von individuellen Patientendatensätzen aus einem externen Patientendatenspeicher, wobei ein Patientendatensatz mindestens eine Angabe zur Identifizierung eines Patienten und mindestens eine Angabe zu einem Gesundheüszustands des Patienten umfasst,
- Auswählen (220) von zwei oder mehreren der Komponentendatensätzen aus einem Komponentenspeicher, der eine Vielzahl von maschinenlesbaren Komponentendatensätzen umfasst, wobei jeder Komponentendatensatz Informationen über einen Abschnitt eines klinischen Behandlungspfads beinhaltet, wobei das Auswählen in Abhängigkeit von den Patientendatensätzen und unter Anwendung einer oder mehrerer Regeln erfolgt, die jeweils eine oder mehrere Bedingungen zur Verknüpfbarkeit von Komponentendatensätzen beinhalten und durch jeweils einen Regeldatensatz aus einem Regelspeicher repräsentiert werden,
- Verknüpfen (230) der ausgewählten Komponentendatensätze zu einem maschinenlesbaren, patientenindividuellen klinischen Behandlungspfad,
- Überprüfen (240) der Erfüllung mindestens eines vordefinierten Prüfkriteriums, wobei das Prüfkriterium das Vorhandensein eines Merkmals des generierten klinischen Behandlungspfads oder eines Patientendatensatzes betrifft.

10. Verfahren (200) nach dem vorhergehenden Anspruch,
**gekennzeichnet durch** den Schritt:
- Wiederholtes Empfangen (210) von individuellen Patientendatensätzen aus einem externen Patientendatenspeicher, wobei die Wiederholung vorzugsweise kontinuierlich, nach vorbestimmten Zeitabschnitten oder an bestimmten Stellen des generierten Behandlungspfads erfolgt oder von einem Nutzer initiiert wird.

11. Verfahren (200) nach einem der beiden vorhergehenden Ansprüche,
**gekennzeichnet durch** den Schritt:
- Wiederholtes Überprüfen (240) der Erfüllung mindestens eines vordefinierten Prüfkriteriums, wobei die Wiederholung vorzugsweise kontinuierlich, nach vorbestimmten Zeitabschnitten oder an bestimmten Stellen des generierten Behandlungspfads erfolgt oder von einem Nutzer initiiert wird.

12. Verfahren (200) nach einem der vorhergehenden Ansprüche 9-11,
**gekennzeichnet durch** den Schritt:
- Verändern (250) des Behandlungspfads oder Ausgeben eines Warnsignals in Abhängigkeit von einem Ergebnis der Überprüfung.

13. Verfahren (200) nach einem der vorhergehenden Ansprüche 9-12,
**gekennzeichnet durch** den Schritt:
- Verändern (260) eines Regeldatensatzes oder eines Komponentendatensatzes in Abhängigkeit von einem Ergebnis der Überprüfung.

14. Verfahren (200) nach einem der vorhergehenden Ansprüche 9-13,
**gekennzeichnet durch** den Schritt:
- Empfangen (270) von Ressourcendatensätzen aus einem weiteren klinischen System, wobei die Ressourcendatensätze Informationen über die Verfügbarkeit von Klinikressourcen beinhalten,
- und wobei der Schritt des Auswählens (220) von zwei oder mehreren der Komponentendatensätze aus einem Komponentenspeicher in Abhängigkeit von den Ressourcendatensätzen erfolgt oder mindestens ein Prüfkriterium das Vorhandensein eines Merkmals eines Ressourcendatensatzes betrifft.

15. Verfahren (200) nach einem der vorhergehenden Ansprüche 9-14,
**gekennzeichnet durch** den Schritt:
- Speichern von Ergebnisdatensätzen über die bei der Durchführung der in generierten Behandlungspfaden repräsentierten Behandlungsschritte, wobei die Ergebnisdatensätze Informationen über Kosten oder Dauer der Behandlungsschritte oder Informationen über Veränderungen von Patientendaten umfasst.
